# EUROPEAN PATENT APPLICATION

(11) **EP 4 290 210 A1**
(43) Date of publication of application: **13.12.2023**
(21) Application number: 22382548.0
(22) Date of filing: 08.06.2022
(51) Int. Cl.: G01N 1/22, G01N 15/06, G01N 15/00

(54) **A DEVICE, A METHOD, AND A COMPUTER PROGRAM, FOR DETECTING AIRBORNE PARTICULATE MATTER IN AEROSOLS**

(71) Applicant: Fundació Institut de Ciències Fotòniques, 08860 Castelldefels (ES); Institució Catalana De Recerca I Estudis Avançats (ICREA), 08010 Barcelona (ES); Universitat Politècnica de Catalunya, 08034 Barcelona (ES)
(72) Inventor: ONGARO, Alfredo, 08860 CASTELLDEFELS (IT); HUSSAIN, Rubaiya, 08860 CASTELLDEFELS (BD); PRUNERI, Valerio, 08860 CASTELLDEFELS (IT); TORNER, Lluís, 08860 CASTELLDEFELS (ES)
(74) Representative: Ponti & Partners, S.L.P

(57) **Abstract**

The present invention relates to a device for detecting airborne particulate matter in aerosols, comprising:
- an air sampler (AS) to collect a sample of airborne particles suspended in air;
- an optical sensor (S);
- control means (C);
- a fluidic apparatus (F) to, under the control of control means (C):
- capture, from the air sampler (AS), and resuspend in a liquid medium, at least part of the airborne particles of the sample; and
- deliver the resuspended airborne particles to the optical sensor (S), which is configured to detect airborne particulate matter in the delivered airborne particles.

The present invention also relates to a method adapted to use the device of the invention, and to a computer program adapted to implement the method of the invention.

## Description

### FIELD OF THE INVENTION

The present invention generally relates, in a first aspect, to a device for detecting airborne particulate matter in aerosols, such as pathogens, allergens and/or other contaminants, and particularly to a device made to allow a quick and simple automatic detection, and also to perform a continuous monitoring.

A second aspect of the present invention relates to a method adapted to use the device of the first aspect of the present invention.

A third aspect of the present invention relates to a computer program adapted to implement the method of the second aspect of the present invention.

### BACKGROUND OF THE INVENTION

The early detection of airborne particulate matter, such as pathogens, in air is critical for various application fields, including environmental safety, bio-hazards, and bio-threats. Current technological approaches focus on air sampling from indoor or outdoor environments, followed by pathogen detection/identification using large equipment in a standard laboratory setting or employing a portable biosensor.

The collection of pathogens in air is performed either by active or passive air samplers. Active samplers use a suction pump or a fan to extract a specified volume of air containing contaminants such as dust particles, allergens, bacteria, and viruses into an air sampler container with collection media. Subsequently, the pathogens in the collected media are measured. Alternatively, the collected air can be directed onto an agar plate for colony formation and subsequent counting after incubation. These methods using active samplers allow both qualitative and quantitative analysis of the sample. Passive air samplers rely on the sedimentation of pathogens on settle plates, i.e., open Petri dishes with non-selective culture media, over a period of time. The plates are incubated for further analysis, such as colony counts for bacteria and plaque assays for viruses. Passive samplers have the tendency to miss small and light pathogens, and they are not quantitative because the volume of sampled air is not known.

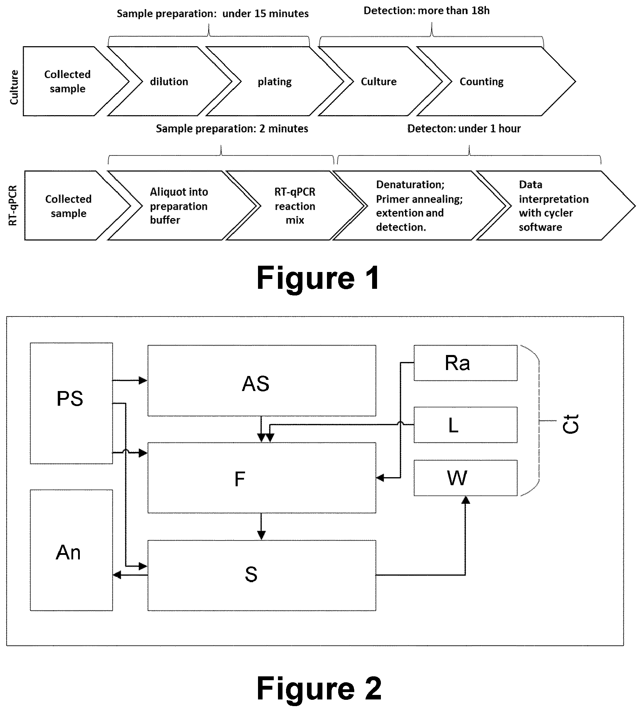

Figure 1 depicts the various approaches for the identification and quantification of airborne pathogens, from sample preparation through detection. Culture-based approaches (top view) rely on the replication of microorganisms in culture to provide viable pathogen concentrations in colony-forming units (CFU) per milliliter. These methods involve diluting the collected sample at various concentrations in order to count colonies; the culture period is determined by the pathogen under study and can range from 24 to 14 days. In the case of viruses, host cells grown on Petri dishes or multi-well plates are infected with various dilutions of the viral sample and covered with a hydrogel substance such as agar. Following an incubation period, the viruses replicate, forming circular regions of damaged cells known as plaques. The plaques represent the number of infectious particles in the sample and the viral load can be calculated in plaque-forming units (PFU) per milliliter.

Apart from the time-consuming colony counting method, which can take from 12h to one week, immunological methods including enzyme-linked immunosorbent assay (ELISA) or nucleic acid-based assays such as real-time/quantitative polymerase chain reaction PCR (qPCR) and reverse-transcriptase PCR (RT-PCR) have been used to detect pathogens due to their high specificity and sensitivity, as well as a shorter detection time (20min - 2h).

One of those immunological methods, particularly that based on real-time quantitative polymerase chain reaction, RT-qPCR, is also shown in Figure 1 (bottom view), and is a molecular technique that involves the following steps: i) an aliquot of the collected sample is dispersed into a preparation buffer; ii) RT-qPCR reaction mix containing appropriate primers, DNA polymerase, etc., are added to the sample followed by iii) denaturation, primer annealing, extension and detection of the fluorescent light; and iv) interpretation of the data to quantify the concentration of the pathogen of interest. The sample preparation involves several manual steps that need to be operated by a specialized technician.

The methods for detecting pathogens in air discussed above are not ideal for continuous monitoring since they involve manual operations to load the collected air sample into a conventional biosensor, which is generally expensive and requires a dedicated laboratory to operate. This adds to the difficulty of properly transporting and storing the samples. To date, several methods and devices, e.g., those described in patents US7633606B2, WO2022024035A1, WO2018210128A1, and CN106967595B, propose integrated systems for air sample collection and pathogen detection, based on lab-on-chip technology. To determine the absence/presence of the target pathogen, current methods use PCR which typically requires multiple modules for nucleic acid extraction, amplification and detection.

Other integrated commercial biosensors in the market, such as Opteev^{™}'s ViraWarn, rely on charge transference technology to detect the electrostatic signature of spiked protein viruses in the air that cause diseases like COVID-19 and influenza and trigger an audible alarm if presence is detected. However, these devices cannot discriminate between different viruses, e.g., COVID-19 and common influenza.

A device for detecting pathogens in aerosols including the features defined in the preamble of claim 1 is known in the prior art, for example from WO2022024035A1, but such device includes the drawbacks mentioned above.

It is therefore necessary to offer an alternative to the state of the art, which covers the gaps mentioned above, particularly by providing a device that does not have the aforementioned drawbacks and limitations and can thus discriminate between different airborne particulate matter, such as pathogens, as well as operate without the multiple modules for nucleic acid extraction, amplification, and detection required in prior art devices.

### SUMMARY OF THE INVENTION

To that end, the present invention relates, in a first aspect, to a device for detecting airborne particulate matter in aerosols, comprising:
- an air sampler configured to collect a sample of airborne particles suspended in air;
- an optical sensor configured to detect airborne particulate matter in at least part of said sample;
- a fluidic apparatus configured at least to deliver at least part of the sample to the optical sensor; and
- control means configured to automatically control the operation of the air sampler, the optical sensor, and the fluidic apparatus.

In contrast to the devices of the prior art, in the one proposed by the first aspect of the present invention the fluidic apparatus is configured and arranged to, under the control of the control means:
- capture, from the air sampler, and resuspend in a liquid medium (such as PBS-Tween), at least part of the above-mentioned airborne particles of the sample; and
- deliver the resuspended airborne particles to said optical sensor;
   and the optical sensor is configured to detect said airborne particulate matter in the delivered airborne particles.

For an embodiment, the device of the first aspect of the present invention is configured for detecting airborne particulate matter in aerosols of ambient air, and the air sampler is an active air sampler.

For another embodiment, complementary or alternative to the above-mentioned embodiment, the device of the first aspect of the present invention is configured for detecting airborne particulate matter in aerosols of exhaled air, for breath analysis.

For a preferred embodiment, the optical sensor is an optical biosensor configured to detect bioparticles included in said airborne particulate matter.

Generally, said bioparticles include one or more pathogens (viruses, bacteria, etc.) and/or one or more allergens (pollen, fungal spores, animal allergens, insect allergens, industrial allergens, etc.), and/or one or more other contaminants.

Depending on the embodiment, the optical biosensor is made to detect one or several types of different allergens or pathogens, such as those of SARS-CoV-2, influenza, Escherichia Coli (E. Coli), etc.

For another embodiment, alternative or complementary to said preferred embodiment, the optical sensor is configured to detect non-biological particles, such as contaminants not including biological material.

Examples of contaminants for any of the above embodiments are dust, dirt, soil, soot, smoke, etc.

The control means comprises, for an embodiment, at least one processor and operatively connected thereto, data storage unit(s) and electric and electronic circuitry, and, for an implementation of that embodiment, the processor is adapted to process an algorithm used to reduce operating time and increase the frequency with which air quality results (i.e., pathogen detection results, whether positive or negative) are generated.

For an embodiment, an alarm unit is comprised by the device of the first aspect of the present invention, operatively connected to the control means and/or to the optical sensor to emit an alarm upon the detection of the airborne particulate matter, such as a pathogen, allergen or other contaminant.

Regarding the optical sensor, depending on the embodiment this is label-free, where the detected signal is generated directly by the interaction of the analysed material with a transducer, or label-based, involving the use of a label and the optical signal is then generated by a colorimetric, fluorescent or luminescent method.

The optical sensor is thus any optical sensor known in the prior art, or variations thereof, that is capable of performing the above defined function, such as a label-free optical sensor based on surface plasmon resonance (SPR), localised surface resonance (LSPR) and surface-enhanced Raman spectroscopy (SERS), spatial light-interference microscopy (SLIM) in combination with neural network or a nano-interferometric sensor.

The optical sensor of the device of the first aspect of the present invention includes all the parts needed for performing the stated detections, which depending on the type of sensor includes parts, modules or units operatively connected with each other, and based on one of more of the following: microelectronics, microelectromechanical systems (MEMSs), micro/nano-technologies, molecular biology parts, biotechnology and chemistry.

For an embodiment, the fluidic apparatus is configured and arranged to, under the control of the control means, label the resuspended airborne particles for the above-mentioned airborne particulate matter, and deliver the labelled resuspended airborne particles to the optical sensor, and wherein the optical sensor is configured to detect the labelled airborne particles. By labelling it has to be understood tagging and/or making luminescent the resuspended airborne particles with a labelled and/or luminescent entity, such as fluorescent antibodies, fluorescent tagged RNA probes, or fluorescent dyes such as Syber Green^{®}.

For an implementation of that embodiment, the fluidic apparatus comprises a reaction chamber to carry out said labelling of the resuspended airborne particles with a reagent.

According to an embodiment, the fluidic apparatus further comprises at least one fluid dispenser, such as a syringe pump, fluidically connectable, under the control of the control means, to the reaction chamber to deliver the resuspended airborne particles, in the liquid medium, to the reaction chamber and to extract therefrom the labelled resuspended airborne particles, and also fluidically connectable to the optical sensor to deliver the same to the optical sensor.

For an embodiment, the fluidic apparatus further comprises a reagent container, containing the above-mentioned reagent, and the at least one fluid dispenser is fluidically connectable, under the control of the control means, to the reagent container to withdraw the reagent therefrom, and to the reaction chamber to deliver the reagent thereinto.

According to an embodiment, the air sampler comprises an air sampler container, and the at least one fluid dispenser is configured for providing, under the control of the control means, the liquid medium to the air sampler container to capture and resuspend in the liquid medium at least part of the airborne particles of the sample contained in the air sampler container.

For an implementation of that embodiment, the air sampler is an active air sampler, such as a cyclone sampler for collecting airborne particles, is connected to the fluidic apparatus via a collection module comprising the above-mentioned air sampler container in the form of a cylinder-conical shaped flask (for example a 2.5 mL flask) and a tube attached to the bottom of the flask.

For an implementation of that embodiment, the fluidic apparatus further comprises a liquid medium container, and the at least one fluid dispenser is fluidically connectable, under the control of the control means, to the liquid medium container to withdraw the liquid medium therefrom.

According to an embodiment, the fluidic apparatus comprises a valvular arrangement automatically controlled by the control means to selectively and fluidically connect at least with part of the air sampler and with the reaction chamber.

According to an implementation of said embodiment, the valvular arrangement is configured to selectively and fluidically connect, under the control of the control means, the at least one fluid dispenser with any of the air sampler container, reaction chamber, reagent container, and liquid medium container.

For a variant of said implementation, the valvular arrangement is also configured to selectively and fluidically connect, under the control of the control means, the at least one fluid dispenser with the optical sensor.

For a different variant of said implementation, the at least one fluid dispenser is configured to directly fluidically connect, under the control of the control means, with the optical sensor, to deliver the labelled airborne particles to the optical sensor without passing through the valvular arrangement.

For an embodiment, the valvular arrangement comprises one valve, particularly a selection valve, with several ports and positions, such as an active rotary valve, although the number of valves is greater than one, for other embodiments.

For an embodiment, the fluid dispenser also comprises at least one valve, to alternatively fluidically connect with the valve(s) of the valvular arrangement and with the optical sensor.

For embodiments for which the valve or valves (of the valvular arrangement or of both the valvular arrangement and the fluid dispenser) are electrically controllable, the control means are electrically connected to those valve or valves and configured to generate and send to the same corresponding control electric signals.

As stated above, the control means also automatically control the operation of the air sampler. Depending on the embodiment, this control is just as simple as activating the same and let it operate continuously, or controlling its activation/deactivation according to different consecutive cycles synchronously with the operation of the fluid dispenser and valvular arrangement.

According to an embodiment, the fluidic apparatus further comprises an air vent, and the valvular arrangement is also configured to selectively and fluidically connect, under the control of the control means, said air vent to the air sampler container to provide a pulsation air flow thereto to aid in the elution and recovery, in the liquid medium, of the captured airborne particles.

For an embodiment, the fluidic apparatus further comprises a waste container fluidically connected or connectable, under the control of the control means, to the optical sensor to receive waste therefrom.

For an embodiment, the at least one fluid dispenser is also configured for providing, as cleaning medium, the above-mentioned liquid medium or another cleaning medium, to clean, under the control of the control means, at least fluid channels of the at least one fluid dispenser, fluid channels of the valvular arrangement, the reaction chamber, and the optical sensor, after use thereof.

According to an embodiment, the device of the first aspect of the present invention is implemented as a compact device integrating at least the air sampler, the optical sensor, and the fluidic apparatus in a common housing, wherein the liquid medium container, reagent container, and waste container are respective removable cartridges removably attached to the common housing or to a support attached thereto.

For an implementation of said embodiment, the control means are configured to automatically control the operation of the air sampler, optical sensor, and fluidic apparatus, continuously according to a sequence of consecutive detection tests, each starting with the air sampling using the air sampler and ending with the waste deliverance to the waste container, said sequence lasting at least until one of the removable cartridges is emptied and thus needs of replacement.

According to an embodiment, the device of the first aspect of the present invention comprises at least a further reaction chamber to label the resuspended airborne particles with the above mentioned reagent or with a further reagent, different to that reagent, to allow the detection of the above mentioned airborne particulate matter or of a further airborne particulate matter that is different to that airborne particulate matter, and wherein the optical sensor is configured to detect the airborne particles labelled with said further reagent.

According to an implementation of that embodiment, the control means are configured to automatically control the operation of the air sampler or of a further air sampler, optical sensor, and fluidic apparatus, continuously according to a further sequence of consecutive detection tests, each detection test starting by the air sampling with the air sampler, or with said further air sampler, and ending with the waste deliverance to the waste container, each sequence lasting at least until one of the removable cartridges is emptied and thus needs of replacement.

According to an embodiment, the control means are configured to automatically control the operation of the air sampler, or air sampler and further air sampler, optical sensor, and fluidic apparatus, to perform the above-mentioned sequence and further sequence of consecutive detection tests at least in part in parallel.

For an embodiment, the device is made to detect naturally luminescent (such as fluorescent) airborne particulate matter, so no labelling is needed. Variants of the above described embodiments are valid to implement corresponding implementations of this embodiment, doing without the labelling actions, and the chambers used therefor (i.e., the reaction chamber(s)), so that the non-labelled resuspended airborne particles including the naturally luminescent airborne particulate matter are delivered by the fluidic apparatus to the optical sensor directly or through an intermediate chamber.

For an embodiment of the device of the first aspect of the present invention, the optical sensor is configured to detect different types of airborne particulate matter, such as different pathogens and/or allergens and/or other contaminants.

For an embodiment, complementary or alternative to the above-mentioned embodiment, the reaction chamber is configured to receive resuspended airborne particles containing different types of airborne particulate matter, naturally luminescent and/or to be labelled with corresponding reagents of the device, under the control of the control means.

For an implementation of those embodiments combined, the fluidic apparatus is configured and arranged to, under the control of the control means, deliver the resuspended airborne particles with the different types of airborne particulate matter (labelled, when not naturally luminescent) to the optical sensor, and wherein the optical sensor is configured to detect the different types of airborne particulate matter simultaneously.

For a preferred embodiment, the fluidic apparatus is a microfluidic apparatus, i.e., an apparatus having microfluidic channels, i.e., fluidic channels having a diameter below 1 mm. The fluidic connections between the fluidic apparatus and the rest of components (containers, chambers, etc.) of the device is also preferably made via microfluidic channels.

However, other embodiments for which the diameter of the fluidic channels and connections is above 1 mm are also embraced by the device of the first aspect of the present invention.

In a second aspect, the present invention relates to a method for detecting airborne particulate matter in aerosols, comprising the following steps:
- collecting, with an air sampler, a sample of airborne particles suspended in air;
- at least delivering, with a fluidic apparatus, at least part of the sample to an optical sensor;
- optically detecting, with said optical sensor, airborne particulate matter in at least part of said sample;
   wherein the operation of the air sampler, the fluidic apparatus, and the optical sensor to perform said steps is automatically controlled.

In contrast to the methods of the prior art, the method of the second aspect of the present invention further comprises:
- automatically controlling the fluidic apparatus to:
   - capture, from the air sampler, and resuspend in a liquid medium, at least part of the airborne particles of said sample; and
   - deliver the airborne particles to the optical sensor;
- and detecting said airborne particulate matter in the delivered airborne particles with the optical sensor.

For an embodiment, the method of the second aspect of the present invention comprises using the device of the first aspect, for any of its embodiments, to detect at least said airborne particulate matter.

According to an implementation of that embodiment, the method of the second aspect of the present invention comprises using the device of the first aspect to perform at least the above-mentioned sequence of consecutive detection tests, each comprising the following consecutive steps:
- an air collection step (c), along a collection time τ_{c} it takes for the air sampler to collect the sample of airborne particles suspended in air;
- a recovery step (r), along a recovery time τᵣ it takes to recover and capture, from the air sampler, and resuspend in the liquid medium, at least part of the airborne particles of the sample;
- a labelling step (I), along a labelling time τₗ it takes to label the resuspended airborne particles; and
- a measuring step, along a measuring time τₘ it takes for the labelled airborne particles to travel to and through the optical sensor and for the optical sensor to detect the labelled airborne particles and calculate its concentration.

The overall time-to-results τₜᵣ can then be considered, fort the above implementation of an embodiment, the sum of air collection time τ_{c}, recovery time τᵣ, labeling time τₗ, and measuring time τₘ. τₗ is usually the largest of all the operational time steps, and it is strictly dependent on the chemical reaction between the tagging material and the particle of interest. When parallel measurements are performed, one can have more frequent results and reduce in this way the time-to-alarm τₐ, i.e., the time between results of subsequent measurements.

The addition of multiple reaction chambers that can be utilized for parallel labelling (as described above) allows to shorten τₐ as it helps overcome large τₗ values. In fact, the device and method of the present invention, for those embodiments including parallel sequences of consecutive detection tests, will give the first result after τₜᵣ. From the second onwards, it will give periodic results every τₐ, with τₐ = τ_{c} + τᵣ assuming that the number of reaction chamber N_{rc} is large enough to satisfy the following condition: N_{rc}≥ [τₗ/(τ_{c} + τᵣ)] and that τₘ ≤ τ_{c} + τᵣ.

The present invention also relates, in a third aspect, to a computer program, including code instructions that when executed on at least one processor of the control means of the device of the first aspect implement the automatic control of the air sampler, optical sensor, and fluidic apparatus, to perform the steps of the method of the second aspect of the invention.

### BRIEF DESCRIPTION OF THE FIGURES

In the following some preferred embodiments of the invention will be described with reference to the enclosed figures. They are provided only for illustration purposes without however limiting the scope of the invention. In accordance with common practice, the components in the figures are drawn to emphasize specific features and they are not drawn to the right scale.
Figure 1 is a schematic representation of prior art methods for aerosol analysis consisting in sample preparation and detection: from top to bottom culture method and RT-qPCR method.
Figure 2 is a schematic representation of the device of the first aspect of the present invention, for an embodiment for which the device is implemented in the form of an integrated automatic online air monitoring device.
Figure 3 is a schematic representation of the collection module or air sampler container of the active air sampler of the device of the first aspect of the present invention, for an embodiment.
Figure 4 is a schematic representation of the fluidic apparatus of the device of the first aspect of the present invention, for an embodiment.
Figure 5 is a schematic representation of the device of the first aspect of the present invention, for an embodiment or configuration for which the fluid dispenser of the fluidic apparatus is connected to the air sampler through the valvular arrangement and directly to the optical sensor, such as an optical biosensor.
Figure 6 is a schematic representation of the device of the first aspect of the present invention, for an embodiment or configuration which differs from that shown in Figure 5 in that the fluid dispenser of the fluidic apparatus is connected to the optical sensor through the valvular arrangement.
Figure 7 is a schematic representation of a possible operation of the device of the first aspect of the present invention, and of the method of the second aspect, for an embodiment, to show that the alarm time interval depends only by the collection and recovery time.
Figure 8: A) Schematic representation of the device of the first aspect of the present invention, for an embodiment or configuration which differs from that shown in Figure 5 in that the fluidic apparatus further comprises an air vent, and in that there are n=4 reaction chambers. B) and C) schematically show different valve positions for the fluid dispenser and selection valve of the valvular arrangement, respectively.
Figure 9 shows proof of concept results obtained with an experimental set-up of the device of the first aspect of the present invention. A) Diagram showing normalized counts of negative control(blank), SARS-COV-2 captured from air, positive control, and air from cleansed environment (after cleaning); B) Diagram showing normalized counts of negative control(blank), E. Coli OP50 captured from air, positive control, and air from cleansed environment (after cleaning).
Figure 10 is a schematic representation of the device of the first aspect of the present invention, for an implementation for which the device is a compact device integrating the different components thereof, including hardware and consumable (liquid medium, reagents, and waste) cartridges.

### DESCRIPTION OF THE PREFERRED EMBODIMENTS

The current invention relates to a fully automated collection and detection device for monitoring indoor/outdoor air for detecting airborne particulate matter, such as pathogens, allergens, and other contaminants in a semi-continuous way.

Figures 2, 4, 5, 6, 8 and 10 show, with different level of detail, the device of the first aspect of the present invention, for different embodiments.

The device comprises at least:
- an active air sampler AS (such as a cyclonic air sampler) configured to collect a sample of airborne particles suspended in air;
- an optical sensor S configured to detect airborne particulate matter, such as pathogens and/or allergen and/or other contaminants in at least part of the sample;
- a fluidic apparatus F, preferably a microfluidic apparatus, configured at least to deliver at least part of the sample to the optical sensor S; and
- control means C configured to automatically control the operation of the active air sampler AS, optical sensor S, and fluidic apparatus F.

The fluidic apparatus F is configured and arranged to, under the control of the control means C:
- capture, from the active air sampler AS, and resuspend in a liquid medium, at least part of said airborne particles of said sample; and
- deliver the resuspended airborne particles to said optical sensor S.

For a preferred embodiment, the optical sensor S is an optical biosensor configured to detect bioparticles, included in the delivered airborne particles, including one or more pathogens and/or one or more allergens and/or one or more other contaminants.

Figure 2 depicts an example block diagram of the device fundamental components identified above, for an embodiment for which the control means C also include a, preferably portable, power source to electrically feed the components of the device, and the device also an antenna An to offer a compact, online air quality monitoring system and airborne particulate matter alarm detector. An alarm unit (not illustrated) is operatively connected to the control means or to the optical sensor S, so that the latter can activate the alarm unit upon the detection of the airborne particulate matter, such as a pathogen or allergen.

For the embodiment of Figure 2, the device comprises, in the form of a set of consumable removable cartridges Ct, a liquid medium container L containing a liquid medium (such as a washing solution) to resuspend the airborne particles, a reagent container Ra containing a reagent to label the resuspended airborne particles, and a waste container W fluidically connected to the optical sensor S to receive waste therefrom (generally, after each detection cycle).

For an embodiment, the optical sensor S is an optical biosensor that is a flow virometry reader (FVR) and the air sampler AS works in active mode. The device of the first aspect of the present invention that integrates, e.g., combines an active air sampler AS, a fluidic apparatus F and a FVR (or another type of optical biosensor S), enables the user to monitor air quality and receive an alert through a communication network (via the antenna An shown in Figure 2).

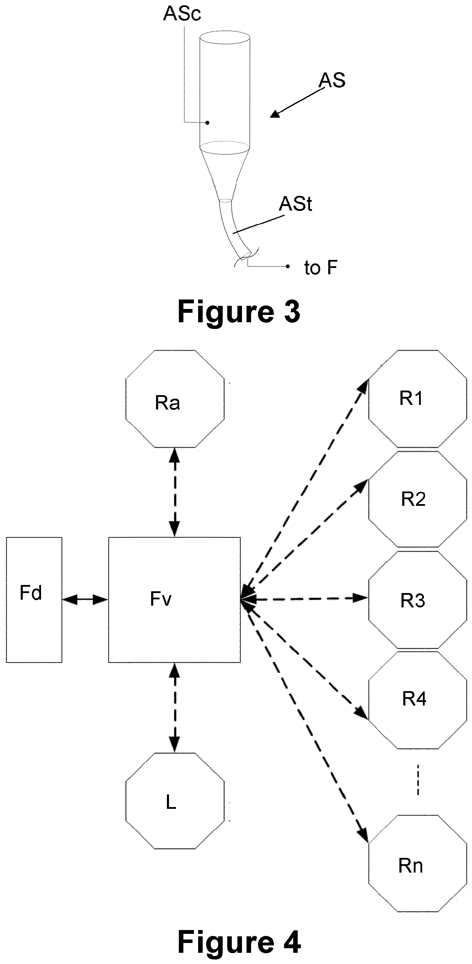

The active air sampler AS, such as a cyclone sampler for collecting airborne particles, is connected to the fluidic apparatus F via a collection module comprising, for example, a 2.5 mL cylinder-conical shaped flask ASc and a tube ASt attached to the bottom of the flask ASc, as shown in Figure 3.

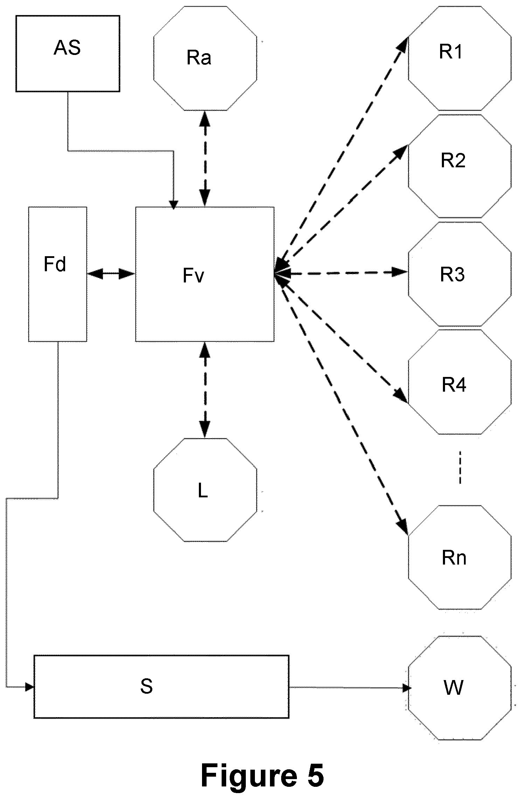

As shown in Figures 4, 5, 6, 8 and 10, the fluidic apparatus F comprises a valvular arrangement Fv, such as a selection valve, and connected thereto a fluid dispenser Fd, such as a syringe pump, one or more consumable reagent cartridges Ra, and one or more wash reagent cartridges L containing washing liquid such as PBS-Tween as the above mentioned liquid medium, and connected to nth reaction chambers R1-Rn (see, for example, Figure 5).

A schematic representation of the fluidic apparatus F is illustrated in Figure 4. This comprises n reaction chambers R1-Rn, the valvular arrangement Fv comprising, for example, an active rotatory one-position nth-port valve. The rotatory valve is connected to a fluid dispenser Fd such as a syringe pump or a peristaltic pump, reagent(s) Ra and a liquid medium or wash container/cartridge L.

Figure 5 shows a possible configuration of the interconnection of the fluidic apparatus F with the air sampler AS, the sensor S and a waste container W. The air sampler AS is connected directly to the selection valve Fv in the schematic, while the sensor S is connected to the fluid dispenser Fd. In this arrangement, the fluid dispenser Fd is equipped with a 3-port, 2-way valve.

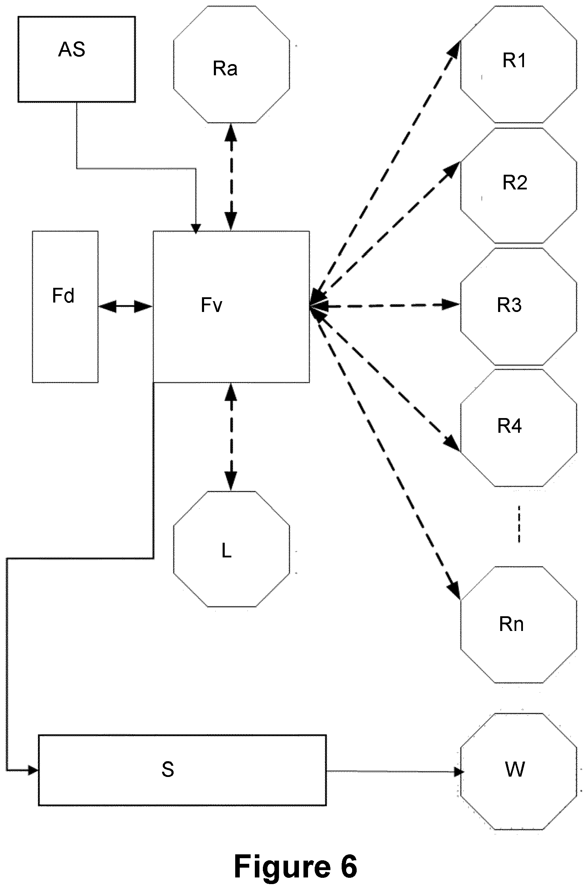

Figure 6 depicts an alternative configuration of the device, in which the air sampler AS and optical sensor S are both directly connected to the selection valve Fv of the fluidic apparatus F.

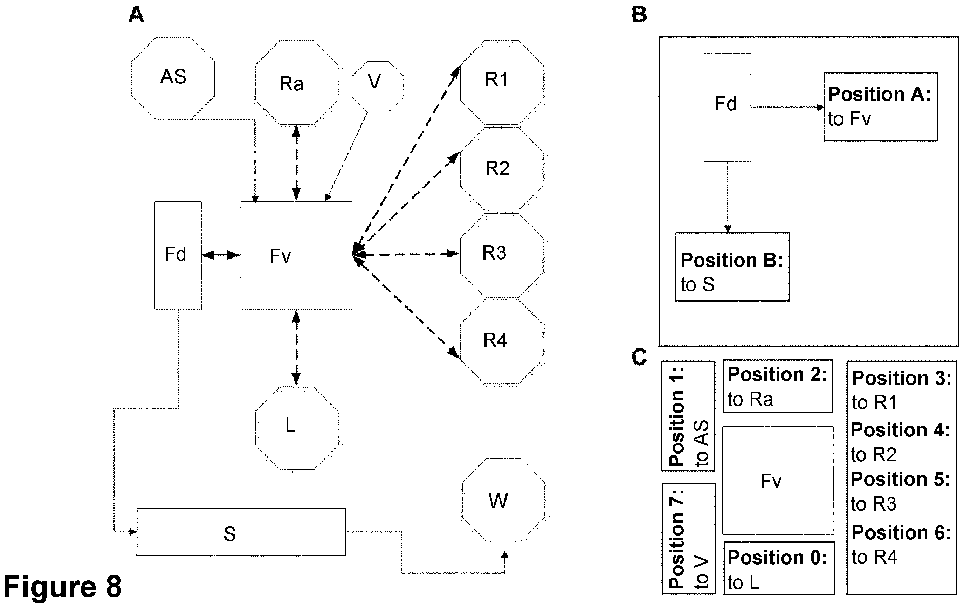

The selection valve Fv enables the washing liquid to be drawn from the reagent cartridge L and delivered to the inside of the air sampler flask ASc. A pulsation step is performed (optionally) to better aid in the elution and recovery of the captured air particles from the flask's inner walls. After being recovered in the washing/liquid medium, the particles are transferred to a reaction chamber R1-Rn. Reagents such as fluorescently labelled antibodies are collected from the reagent cartridge Ra and delivered to the reaction chamber R1-Rn, where a pulsation flow (or another type of mixing mechanism) is used to mix the target particles with the tagging molecules. Through the selection valve Fv, all of these stages are carried out automatically, controlled by the control means C. Finally, using the fluid dispenser Fd (for the embodiments of Figure 5 and 8) or the valvular arrangement Fv (for the embodiment of Figure 6), the labelled sample is transferred to the sensor S, which, generally optically interrogates the sample at an interrogation location of a microfluidic channel.

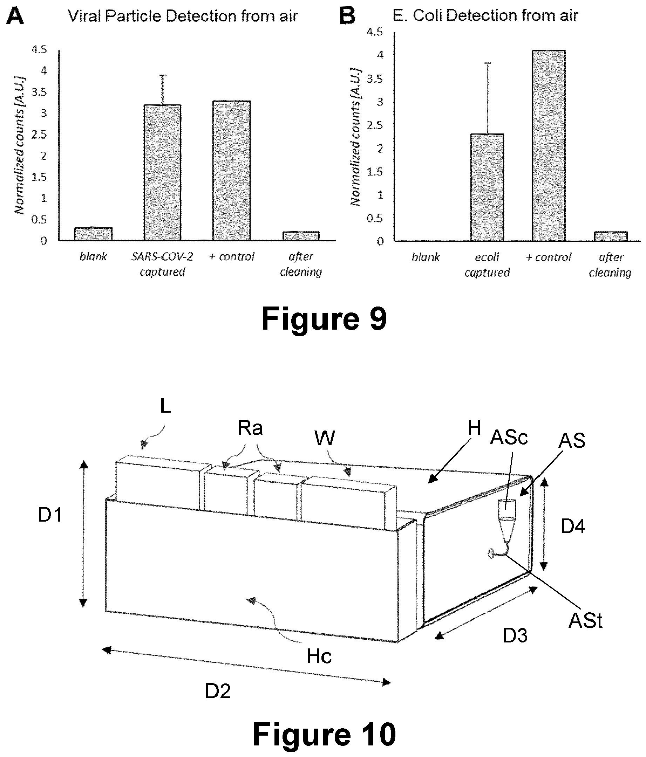

Figure 10 a schematic representation of the device of the first aspect of the present invention, for the implementation for which the device is a compact device integrating the different components thereof, particularly an active air sampler AS, including the collection flask ASc and tube ASt, liquid medium cartridge L, reagent cartridges Ra (containing the same or, in case different airborne particulate matter are to be detected, different reagents), waste cartridge W. The cartridges L, Ra, W are removably attached to a support or pocket-like housing Hc attached to a common housing H housing the rest of components not illustrated in the Figure (control means C, fluid dispenser Fd, valvular arrangement Fv, and optical sensor S).

A device following the design of Figure 10 was built for performing 144 measurements in a single day, so that the removable cartridges would have to be filled or interchanged by filled ones only at the end of each day. The built device had the following dimensions: D1 = 16 cm, D2 = 30 cm, D3 = 25 cm, D4 = 13.5 cm, and the cartridges had the following capacity: liquid medium (or wash liquid) cartridge L = approx. 2 litres, reagent cartridges Ra = approx. 250 mL each reagent cartridge (or 500 mL when only one reagent cartridge is used), and waste cartridge W = approx. 2 litres.

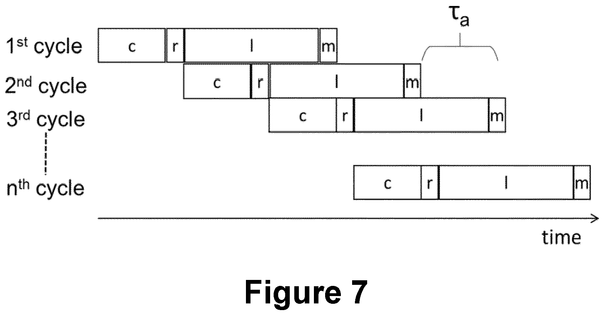

Figure 7 depicts how the addition of multiple reaction chambers can be utilized for parallel labelling allowing to shorten τₐ until reaching the minimum possible value equal to τ_{c} + τᵣ. To shorten it further one would have to use two or more devices of the first aspect of the present invention working in parallel. Each box represents one of the automated device's operating steps, performed according to an embodiment of the method of the second aspect of the present invention, and which are explained as:
C = collection, which is the time it takes for the air sampler AS to collect aerosols and other particles in the air;
R = recovery, which is the time it takes to wash (with the liquid medium) and recover the trapped air particles in the collection flask ASc;
L = labelling, which is the time it takes to label the sample;
M = measuring, which is the time it takes for the sample to travel through the sensor S and the software to calculate the particle concentration.

Each cycle is made up of the following components: collection time τ_{c}, recovery time τᵣ, labeling time τₗ, and measuring time τₘ. τₐ denotes the time-to-alarm interval of subsequent measurements for the presence of airborne particulate matter in the air.

Figure 8A shows an example of the device of the first aspect of the present invention, for an embodiment or configuration which differs from that shown in Figure 5 in that the fluidic apparatus F further comprises an air vent V, and in that there are n=4 reaction chambers.

Figure 8B lists the different positions of the 3 port 2-way valve of the fluid dispenser Fd. Position A fluidically connects the fluid dispenser Fd to the selection valve Fv and position B fluidically connects the fluid dispenser to the optical sensor S.

In Figure 8C, the possible positions of the 9 port active rotary valve Fv are illustrated. Position 0 fluidically connects the selection valve Fv to the wash cartridge L; position 1 fluidically connects the selection valve Fv to the air sampler AS; position 2 fluidically connects the selection valve Fv to the reagent cartridge Ra, Position 3 fluidically connects the selection valve Fv to reaction chamber 1; position 4 fluidically connects the selection valve Fv to reaction chamber 2; position 5 fluidically connects the selection valve Fv to reaction chamber 3; position 6 fluidically connects the selection valve Fv to reaction chamber 4; position 7 fluidically connects the selection valve Fv to air vent V.

A proof of concept experimental set-up has been built by the present inventors to provide experimental evidence supporting the invention disclosure.

The experimental set-up consists of a custom-made hermetic box of 36 L (30cmx30cmx40xm) with the scope to create a controlled closed environment. The box is equipped with an input hole to which a commercially available nebulizer is connected, and a door to allow the placement of the air sampler and the cone trapping the sampled particles. The air sampler is a commercially available, active, portable, cyclonic air sampler that operates at a flow rate of 50 L/minutes. The biosensor employed for the detection of the collected particles is a custom-build small form factor flow virometer reader (FVR). The FVR combines sample flow in a straight microfluidic channel at a flowrate of 1 mL/minutes using an automatic syringe pump (fluidic control), and a blue laser interrogation to detect the fluorescence light emitted by the targeted pathogen or allergen passing through the field of view.

Figure 9 shows proof of concept results obtained with the experimental set-up described above. To determine the feasibility of capturing and detecting viruses, an environment containing heat-inactivated SARS-CoV-2 viral particles dispersed in aerosols was created. First, a 2 mL solution of 50,000 viral copies per milliliter in PBS was prepared by serial dilution from a commercially available SARS-CoV-2 sample from ATTC, with a starting concentration of 10⁸copies/mL. The prepared mock sample was nebulized inside the hermetic box to generate a concentration of 2.7 viral copies/mL in air. The air is collected using the cyclonic air sampler for 2 minutes, and the trapped viral particles are recovered from the inner surface of the cone by washing it with 2 mL of PBS. Once the sample has been collected, it is labelled by adding 50 ng/mL fluorescent labelled antibodies against SARS-CoV-2 and incubated for 20 minutes.

The labelled sample is then pumped through the FVR at 1 mL/min and the fluorescent events are detected. A series of controls were performed. As negative control, air collected prior to virus nebulization has been labelled and measured. As positive control, a 1 mL mock sample containing 50,000 viral copies per milliliters in PBS has been labelled, and measured. As an additional control, once the air containing the nebulized virus was collected, the hermetic box, and instruments have been cleaned using UV light and Ethanol. Then, the air from the cleaned box was collected, recovered, labelled, and measured. Figure 9A shows the experimental results of the detection of SARS-CoV-2 from air. On the y-axis, the normalized counts [A.U.] of the fluorescent events are reported. Normalized counts here represent the counted events of each measured sample divided by the average counts of the fluorescent antibodies used. On the x-axis, controls and captured SARS-CoV-2 are reported. As can be seen, there is a statistically significant increase of the normalized counts of the SARS-CoV-2 captured samples with respect to the blank, and the signal of the captured virus is not different from the positive control, suggesting that most of the nebulized virus has been captured. The normalized counts of the collected air after the device was cleaned dropped to the level of the blank. This suggests that the proposed device can detect viruses in air and check for the air quality with respect to pathogen presence.

To determine the feasibility of capturing and detecting bacteria, the same experiment described above has been performed using Escherichia Coli (E. Coli), as example. E. Coli OP50 at a concentration of 1,000 CFU/mL was nebulized inside the hermetic box to create an environment containing 55 CFU/100 mL of bacteria in the air. Then the bacteria dispersed in air has been captured, labelled using fluorescent-labelled E. Coli antibodies, and measured (Figure 9B). The device proved to be effective in the detection of E. coli from air.

An example of operation of the device of the first aspect of the present invention, and also of the method of the second aspect, is described below. In the following example the device of the embodiment of Figures 8A, 8B and 8C is used, and different cycles performed in part in parallel, as shown in Figure 7, are performed.

### Example:

### 1^{st} cycle:

1. Air sampler AS starts collecting.
2. Fluid dispenser Fd position A.
3. Selection valve Fv position 0, for a specific time, such as 2 seconds.
4. Fluid dispenser Fd withdraws washing liquid from the wash cartridge L, for example during 6 seconds for 1 ml at 10 ml/min.
5. Fluid dispenser Fd filled with wash liquid, for example for 6 sec.
6. Selection valve Fv position 1, for example for 2 seconds.
7. Air sampler AS stops collection.
8. Fluid dispenser Fd delivers wash liquid to the collection tube ASt, and thus to the air sample flask ASc, for example for 6 seconds.
9. Selection valve Fv position 7, for example for 2 seconds.
10. Fluid dispenser Fd withdraws air, for example for 6 seconds.
11. Selection valve Fv position 1, for example for 2 seconds.
12. Fluid dispenser Fd delivers air to collection tube ASc, and thus to the air sample flask ASc, to create pulsation flow, for example for 6 seconds.
13. Fluid dispenser Fd withdraws recovered air particle in washing liquid, for example for 6 seconds.
14. Air sampler AS starts collecting.
15. Selection valve Fv position 3, for example for 2 seconds.
16. Fluid dispenser Fd delivers sample 1, i.e., recovered air particle in washing liquid, to the reaction chamber R1, for example for 6 seconds.
17. Selection valve Fv position 2, for example for 2 seconds.
18. Fluid dispenser Fd withdraws reagent from Ra, for example for 6 seconds.
19. Selection valve Fv position 3, for example for 2 seconds.
20. Fluid dispenser Fd delivers reagent to the filled reaction chamber R1, for example for 6 seconds.

### 2^{nd} cycle:

21. Selection Valve Fv position 0.
22. Air sampler AS stops collection.
23. Fluid dispenser Fd withdraws washing liquid from wash cartridge L.
24. Fluid dispenser Fd filled with wash liquid.
25. Selection valve Fv position 1.
26. Fluid dispenser Fd delivers wash liquid to the collection tube ASc, and thus to the air sample flask ASc.
27. Selection valve Fv position 7.
28. Fluid dispenser Fd withdraws air.
29. Selection valve Fv position 1.
30. Fluid dispenser Fd delivers air to collection tube ASc, and thus to the air sample flask ASc, to create pulsation flow.
31. Fluid dispenser Fd withdraws recovered air particle in washing liquid.
32. Air sampler AS starts collecting.
33. Selection valve Fv position 4.
34. Fluid dispenser Fd delivers sample 2, i.e., recovered air particle in washing liquid, to the reaction chamber R2.
35. Selection valve Fv position 2.
36. Fluid dispenser Fd withdraws reagent from Ra.
37. Selection valve Fv position 4.
38. Air sampler AS stops collection.
39. Fluid dispenser Fd delivers reagent to fill reaction chamber R2.
40. Selection valve Fv position 3.
41. Fluid dispenser Fd withdraws labelled sample 1 from R1.
42. Fluid dispenser Fd position B.
43. Fluid dispenser Fd delivers labelled sample 1 for measuring to the sensor S.

### 3^{rd} cycle:

44. Selection valve Fv position 0.
45. Air sampler AS stops collection.
46. Fluid dispenser Fd withdraws washing liquid from wash cartridge L.
47. Fluid dispenser Fd filled with wash liquid.
48. Selection valve Fv position 1.
49. Fluid dispenser Fd delivers liquid to the collection tube ASt, and thus to the air sample flask ASc.
50. Selection valve Fv position 7.
51. Fluid dispenser Fd withdraws air.
52. Selection valve Fv position 1.
53. Fluid dispenser Fd delivers air to collection tube ASt, and thus to the air sample flask ASc, to create pulsation flow.
54. Fluid dispenser Fd withdraws recovered air particle in washing liquid.
55. Air sampler AS starts collecting.
56. Selection valve Fv position 5.
57. Fluid dispenser Fd delivers sample 3, i.e., recovered air particle in washing liquid, to reaction chamber R3.
58. Selection valve Fv position 2.
59. Fluid dispenser Fd withdraws reagent from Ra.
60. Selection valve Fv position 5
61. Air sampler AS stops collection.
62. Fluid dispenser Fd delivers reagent to fill reaction chamber R3.
63. Selection valve Fv position 4.
64. Fluid dispenser Fd withdraws labelled sample 2.
65. Fluid dispenser Fd position B.
66. Fluid dispenser delivers labelled sample 2 for measuring to the sensor S.

The times in seconds indicated in Cycle 1 above are provided just as exemplary, and can vary, for example, depending on the type of reaction and type of sample to be labelled in the reaction chamber. Similar times can be used for subsequent cycles.

Some example use cases of the device of the first aspect of the present invention are described below.

### Case 1: Single pathogen detection:

Considering the case of detecting a single pathogen, for e.g., SARS-CoV-2, for which the collection time τ_{c} is 10 minutes, the recovery time τᵣ is 1 minute, and the labelling time τₗ is 20 minutes. For this particular case, the required number of reaction chambers N_{rc}≥ 2 i.e., at least two reaction chambers. With respect to Figure 7, Cycle 1 starts with sample collection, from c1= 0 lasting for 10 minutes i.e., till c1=10. The recovery time of 1 minute (r1=11) includes changing valve positions, withdrawing wash liquid from the wash cartridge L, delivering to the collection tube ASt, and thus air sample flask ASc, to elute the particles and finally delivering sample 1 to reaction chamber R1. The next step is labelling sample 1 for 20 minutes (l1=31). This step also includes the time taken by the fluid dispenser Fd to withdraw the reagent from the reagent cartridge Ra and deliver it to reaction chamber R1. The measurement time is 2 minutes which also includes the time for cleaning the microfluidic apparatus F after each sample measurement. Thus, sample 1 measurement is completed after a total time of 33 minutes (m1=33). Cycle 2 starts immediately after the recovery time of sample 1 i.e., c2 = 11. In the same way as the previous cycle, sample 2 is then eluted after collection, labelled and delivered to reaction chamber R2. It is then measured after a total time of 43 minutes (m2=44). Thus, using multiple reaction chambers time-to-alarm, τₐ is effectively shortened to 11 minutes. A cleaning cycle has to be included to clean the selection valve tubes and the fluid dispenser Fd carrying the fluorescent reagents after they are used to deliver the reagent to a reaction chamber. The table shows the time for each cycle if three reaction chambers are used.

| | Cycle 1 (minutes) | Cycle 2 (minutes) | Cycle 3 (minutes) |
|---|---|---|---|
| collection = 10 min | c1= 10 | c2= 11 | c3 = 22 |
| recovery = 1 min | r1= 11 | r2= 22 | r3= 33 |
| labelling = 20 min | l1=31 | l2=42 | l3=53 |
| measuring = 2 min | m1= 33 | m2= 44 | m3= 55 |

### Case 2: Single pathogen detection with specific probe that fluorescent only when binding occurs:

Same as Case 1 with the exception that the reagent used to label the sample is fluorescent only when binding occurs between the antibodies and the pathogen of interest. Therefore, the cleaning procedure is carried out only when a signal is detected after the sample has been measured.

### Case 3: Two different pathogens, same excitation different emission wavelength:

In this example, consider two different pathogens to be detected with only one reagent cartridge Ra. The fluorescent antibodies/probes/dyes used in this case have the same excitation wavelength but different emission wavelengths. The labelling time is also the same for both. If we assume the same collection, recovery, labelling time as Case 1, then, with at least two reaction chambers (N_{rc}≥ 2) per pathogen, the time-to-alarm τₐ is the same as before i.e., 11 minutes.

### Case 4: Two different pathogens, different labelling time, excitation and emission wavelength:

Same as in Case 3 with the exception that the excitation wavelength of the fluorescent antibodies/probes/dyes and the labelling time for each pathogen are different. If the same collection and recovery time as Case 1 is assumed, but different labelling time, then the minimum number of reaction chamber needed to shorten the time-to-alarm is the sum of the minimum number of reaction chambers to label pathogen 1 and pathogen 2.

| Pathogen 1 | Pathogen 2 |
|---|---|
| τ_{c}= 10 minutes, | τ_{c}= 10 minutes, |
| τᵣ= 1 minute | τᵣ= 1 minute |
| τₗ= 20 minutes | τₗ= 30 minutes |
| N_{rc}≥ 2 | N_{rc}≥ 3 |

In this case the minimum number of reaction chambers needed is 5, which will result on a time-to-alarm of 11 minutes.

A person skilled in the art could introduce changes and modifications in the embodiments described without departing from the scope of the invention as it is defined in the attached claims.

## Claims

1. A device for detecting airborne particulate matter in aerosols, comprising:
- an air sampler (AS) configured to collect a sample of airborne particles suspended in air;
- an optical sensor (S) configured to detect airborne particulate matter in at least part of said sample;
- a fluidic apparatus (F) configured at least to deliver at least part of said sample to said optical sensor (S); and
- control means (C) configured to automatically control the operation of said air sampler (AS), said optical sensor (S), and said fluidic apparatus (F);
**characterized in that** said fluidic apparatus (F) is configured and arranged to, under the control of said control means (C):
- capture, from said air sampler (AS), and resuspend in a liquid medium, at least part of said airborne particles of said sample; and
- deliver the resuspended airborne particles to said optical sensor (S);
and **in that** the optical sensor (S) is configured to detect said airborne particulate matter in the delivered airborne particles.

2. The device of claim 1, wherein said optical sensor (S) is an optical biosensor configured to detect bioparticles, included in said airborne particulate matter, including one or more pathogens and/or one or more allergens and/or one or more other contaminants.

3. The device of claim 1 or 2, wherein said fluidic apparatus (F) is configured and arranged to, under the control of said control means (C), label the resuspended airborne particles for said airborne particulate matter, and deliver the labelled resuspended airborne particles to the optical sensor (S), and wherein the optical sensor (S) is configured to detect the labelled airborne particles.

4. The device of claim 3, wherein the fluidic apparatus (F) comprises a reaction chamber (R1) to carry out said labelling of the resuspended airborne particles with a reagent; and wherein the fluidic apparatus (F) further comprises at least one fluid dispenser (Fd) fluidically connectable, under the control of said control means (C), to said reaction chamber (R1) to deliver the resuspended airborne particles, in the liquid medium, to said reaction chamber (R1) and to extract therefrom the labelled resuspended airborne particles, and also fluidically connectable to the optical sensor (S) to deliver the same to the optical sensor (S).

5. The device of claim 4, wherein:
- the fluidic apparatus (F) further comprises a reagent container (Ra) containing said reagent, and wherein said at least one fluid dispenser (Fd) is fluidically connectable, under the control of said control means (C), to said reagent container (Ra) to withdraw said reagent therefrom, and to said reaction chamber (R1) to deliver the reagent thereinto; and/or
- the air sampler (AS) comprises an air sampler container (ASc), and wherein said at least one fluid dispenser (Fd) is configured for providing, under the control of said control means (C), said liquid medium to said air sampler container (ASc) to capture and resuspend in the liquid medium at least part of the airborne particles of the sample contained in the air sampler container (ASc); and/or
- the fluidic apparatus (F) further comprises a liquid medium container (L), and wherein the at least one fluid dispenser (Fd) is fluidically connectable, under the control of said control means (C), to said liquid medium container (L) to withdraw said liquid medium therefrom.

6. The device of claim 4 or 5, wherein the fluidic apparatus (F) comprises a valvular arrangement (Fv) automatically controlled by the control means (C) to selectively and fluidically connect at least with part of said air sampler (AS) and with said reaction chamber (R1).

7. The device of claim 6 when depending on claim 5, wherein said valvular arrangement (Fv) is configured to selectively and fluidically connect, under the control of said control means (C), said at least one fluid dispenser (Fd) with any of said air sampler container (ASc), reaction chamber (R1), reagent container, and liquid medium container (L).

8. The device of claim 7, wherein:
- said valvular arrangement (Fv) is also configured to selectively and fluidically connect, under the control of said control means (C), said at least one fluid dispenser (Fd) with said optical sensor (S); or
- said at least one fluid dispenser (Fd) is configured to directly fluidically connect, under the control of said control means (C), with said optical sensor (S), to deliver the labelled airborne particles to the optical sensor (S) without passing through the valvular arrangement (Fv).

9. The device of claim 7 or 8, wherein the fluidic apparatus (F) further comprises:
- an air vent (V), and wherein the valvular arrangement (Fv) is also configured to selectively and fluidically connect, under the control of said control means (C), said air vent (V) to the air sampler container (ASc) to provide a pulsation air flow thereto to aid in the elution and recovery, in the liquid medium, of the captured airborne particles; and/or
- a waste container (W) fluidically connected or connectable, under the control of said control means (C), to the optical sensor (S) to receive waste therefrom.

10. The device of claim 9, implemented as a compact device integrating at least said air sampler (AS), said optical sensor (S), and said fluidic apparatus (F) in a common housing (H), wherein said liquid medium container (L), reagent container (Ra), and waste container (W) are respective removable cartridges removably attached to said common housing (H) or to a support (Hc) attached thereto.

11. The device of claim 10, wherein the control means (C) are configured to automatically control the operation of the air sampler (AS), fluidic apparatus (F), and optical sensor (S), continuously according to a sequence of consecutive detection tests, each starting by the air sampling with the air sampler (AS) and ending with the waste deliverance to the waste container (W), said sequence lasting at least until one of said removable cartridges (L, Ra, W) is emptied and thus needs of replacement.

12. The device of any of claims 4 to 11, comprising at least a further reaction chamber (R2, R3, R4, R5, ....Rn) to label the resuspended airborne particles with said reagent or with a further reagent, different to said reagent, to allow the detection of said airborne particulate matter, or of a further airborne particulate matter that is different to said airborne particulate matter, and wherein the optical sensor (S) is configured to detect the airborne particles labelled with said further reagent, and wherein the control means (C) are configured to automatically control the operation of the air sampler (AS) or of a further air sampler (AS), fluidic apparatus (F), and optical sensor (S), continuously according to a further sequence of consecutive detection tests, each detection test starting by the air sampling with the air sampler (AS), or with said further air sampler (AS), and ending with the waste deliverance to the waste container (W), each sequence lasting at least until one of the removable cartridges (L, Ra, W) is emptied and thus needs of replacement.

13. The device of claim 12, wherein the control means (C) are configured to automatically control the operation of the air sampler (AS), or air sampler (AS) and further air sampler (AS), fluidic apparatus (F), and optical sensor (S), to perform said sequence and said further sequence of consecutive detection tests at least in part in parallel.

14. A method for detecting airborne particulate matter in aerosols, comprising the following steps:
- collecting, with an air sampler (AS), a sample of airborne particles suspended in air;
- at least delivering, with a fluidic apparatus (F), at least part of said sample to an optical sensor (S);
- optically detecting, with said optical sensor (S), airborne particulate matter in at least part of said sample
wherein the operation of said air sampler (AS), said fluidic apparatus (F), and said optical sensor (S) to perform said steps is automatically controlled;
**characterized in that** the method further comprises:
- automatically controlling said fluidic apparatus (F) to:
- capture, from said air sampler (AS), and resuspend in a liquid medium, at least part of said airborne particles of said sample; and
- deliver the resuspended airborne particles to said optical sensor (S);
- and detecting said airborne particulate matter in the delivered airborne particles with the optical sensor (S).

15. A computer program, including code instructions that when executed on at least one processor of the control means (C) of the device of any of claims 1 to 13 implement the automatic control of the air sampler (AS), optical sensor (S), and fluidic apparatus (F), to perform the steps of the method of claim 14, to detect at least said airborne particulate matter.
